# EUROPÄISCHE PATENTSCHRIFT

(11) **EP 1 115 708 B1**
(45) Veröffentlichungstag und Bekanntmachung des Hinweises auf die Patenterteilung: **14.04.2004**
(21) Anmeldenummer: 99948842.2
(22) Anmeldetag: 20.09.1999
(51) Int. Cl.: C07D 221/16, A61K 31/435, C07D 405/04, C07D 221/18

(54) **AMINOALKYL-3,4-DIHYDROCHINOLIN-DERIVATE ALS NO SYNTHASE INHIBITOREN**
AMINOALKYL-3,4-DIHYDROQUINOLINE DERIVATES AS NO-SYNTHASE INHIBITORS
DERIVES D'AMINOALKYL-3,4-DIHYDROQUINOLEINE EN TANT QU'INHIBITEURS DE NO SYNTHASE

(30) Priorität: 24.09.1998 DE 19845830
(43) Veröffentlichungstag der Anmeldung: 18.07.2001
(73) Patentinhaber: Schering Aktiengesellschaft, 13353 Berlin (DE)
(72) Erfinder: JAROCH, Stefan, D-10625 Berlin (DE); REHWINKEL, Hartmut, D-12051 Berlin (DE); HÖLSCHER, Peter, D-10559 Berlin (DE); SÜLZLE, Detlev, D-10589 Berlin (DE); HILLMANN, Margrit, D-13437 Berlin (DE); BURTON, Gerardine, Anne, D-13591 Berlin (DE); MCDONALD, Fiona, Mcdougall, D-14055 Berlin (DE)
(86) Internationale Anmeldenummer: PCT/EP1999/007091
(87) Internationale Veröffentlichungsnummer: WO 2000/017167

(56) Entgegenhaltungen:
- WO-A-96/14844
- WO-A-99/41240

## Beschreibung

Die Erfindung betrifft 3,4-Dihydrochinolin-Derivate, ein Verfahren zu ihrer Herstellung und ihre Verwendung in Arzneimitteln.

In menschlichen Zellen existieren mindestens drei Formen von Stickstoffmonoxid-Synthasen, die Arginin in Stickstoffmonoxid (NO) und Citrullin überführen. Es wurden zwei konstitutive NO-Synthasen (NOS) identifiziert, die als Calzium / Calmodulin abhängige Enzyme im Gehirn (ncNOS oder NOS 1) bzw. im Endothel (ecNOS oder NOS 3) vorhanden sind. Eine weitere Isoform ist die induzierbare NOS (iNOS oder NOS 2), die ein praktisch Ca⁺⁺ unabhängiges Enzym ist und nach Aktivierung unterschiedlicher Zellen durch Endotoxin oder andere Stoffe induziert wird.

NOS-Inhibitoren und insbesondere spezifische Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden

Eine Reihe von Reviews informiert über Wirkung und Inhibitoren von NO-Synthasen. Genannt seien beispielsweise: Drugs 1998, **1**, 321 oder Current Pharmac. Design 1997, **3**, 447.

Als NOS-Inhibitoren sind unterschiedliche Verbindungen bekannt. Beispielsweise werden Argininderivate, Aminopyridine, cyclische Amidinderivate, Phenylimidazole und andere beschrieben. Cyclische Amidine werden in WO 96/14844 offenbart.

Es wurde nun gefunden, daß die erfindungsgemäß substituierten Heterocyclen gegenüber bekannten Verbindungen besonders vorteilhaft als Arzneimittel verwendet werden können.

Die Erfindung betrifft die Verbindungen der Formel I, deren tautomere und isomere Formen und Salze 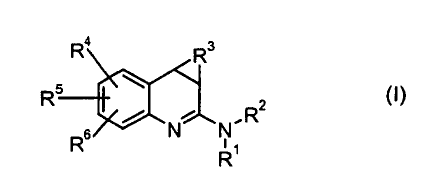 worin die Substituenten folgende Bedeutung haben:
R¹ und R² bedeuten Wasserstoff,
R³ bedeutet:
   einen C₁₋₅-Alkylenrest,
R⁴ bedeutet:
C₁₋₄-Alkyl, substituiert mit NR¹⁴R¹⁵ oder
R⁴ und R⁵ bilden gemeinsam mit 2 benachbarten Kohlenstoffatomen einen fünfoder sechsgliedrigen Carbocyclus, der mit NR¹⁴R¹⁵ substituiert sein kann,
R⁵ und R⁶ bedeuten unabhängig voneinander:
   a) Wasserstoff,
   b) Halogen,
   c) OR⁷,
   d) C₁₋₄-Alkyl
   e) CF₃,
   f) OCF₃,
R⁷ bedeutet:
   a) Wasserstoff,
   b) C₁₋₆-Alkyl,
   c) C6-10-Aryl, das gegebenenfalls mit Halogen oder C₁₋₄-Alkyl substituiert ist,
R²⁰ bedeutet:
   a) C₁₋₆-Alkyl,
   b) C6-10-Aryl, das gegebenenfalls mit Halogen oder C₁₋₄-Alkyl substituiert ist,
R¹⁴ und R¹⁵ bedeuten unabhängig voneinander:
   a) Wasserstoff
   b) CO₂R²⁰
   c) C₁₋₆-Alkyl, das gegebenfalls substituiert ist mit Halogen, Hydroxy, C₁₋₄-Alkoxy, Nitro, Amino, C₁₋₆-Alkyl, Trifluormethyl, Carboxyl, Cyano, Carboxamido, C₃₋₇-Cycloalkyl, Indanyl, 1,2,3,4-Tetrahydronaphthyl, C₆₋₁₀-Aryl, 5- oder 6gliedrigen Heteroaryl mit 1 - 4 Stickstoff-, Sauerstoffoder Schwefelatomen, das mit Benzol anelliert sein kann, wobei der Arylund der Heteroarylrest mit Halogen, Hydroxy, C₁₋₄-Alkoxy, C₁₋₄-Alkyl, CF₃, NO₂, NH₂, N(C₁₋₄-Alkyl)₂ oder Carboxyl substituiert sein können,
   oder
   R¹⁴ und R¹⁵ bilden gemeinsam mit dem Stickstoffatom einen 5 - 7gliedrigen gesättigten Heterocyclus, der ein weiteres Sauerstoff-, Stickstoff- oder Schwefelatom enthalten und mit C₁₋₄-Alkyl oder einem gegebenenfalls mit Halogen substituierten Phenyl-, Benzyl- oder Benzoyl-Rest substituiert sein kann oder einen ungesättigten 5gliedrigen Heterocyclus, der 1 - 3 N-Atome enthalten und substituiert sein kann mit Phenyl, C₁₋₄-Alkyl, Halogen oder CH₂-OH.

Die Verbindungen der Formel können als Tautomere, Stereoisomere oder geometrische Isomere vorliegen. Die Erfindung umfaßt auch alle möglichen Isomere wie E- und Z-Isomere, S- und R-Enantiomere, cis- und trans-Diastereomere, Racemate und Gemische derselben einschließlich der tautomeren Verbindungen der Formel Ia und Ib (für R² = Wasserstoff).

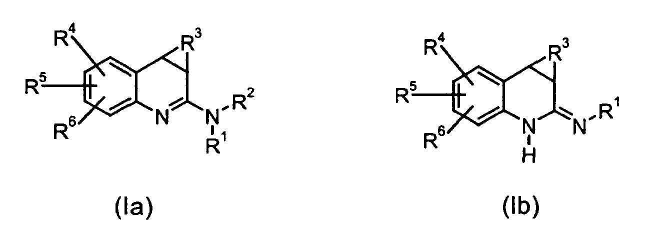

Die physiologisch verträglichen Salze können mit anorganischen und organischen Säuren gebildet werden wie beispielsweise Oxalsäure, Milchsäure, Zitronensäure, Fumarsäure, Essigsäure, Maleinsäure, Weinsäure, Phosphorsäure, Salzsäure, Bromwasserstoffsäure, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige und verzweigte Alkylgruppe wie z.B. Methyl, Ethyl, Propyl, Isopropyl, n-Butyl, sek-Butyl, tert-Butyl, n-Pentyl, sek-Pentyl, tert-Pentyl, Neopentyl, n-Hexyl, sek-Hexyl, Heptyl, Octyl.

Unter Cycloalkyl ist jeweils Cyclopropyl, Cyclobutyl, Cyclopentyl, Cyclohexyl und Cycloheptyl zu verstehen.

Halogen bedeutet jeweils Fluor. Chlor. Brom und Jod.

Unter Aryl ist jeweils Naphthyl und insbesondere Phenyl zu verstehen, die einbis dreifach gleich oder verschieden in beliebiger Position substituiert sein können.

Der Hetarylrest kann jeweils einen ankondensierten Benzolring enthalten und ein- bis dreifach gleich oder verschieden substituiert sein. Beispielsweise sind die folgenden 5- und 6-Ringheteroaromaten geeignet:

Imidazol, Indol, Isooxazal, Isothiazol, Furan. Oxadiazol. Oxazol, Pyrazin, Pyridazin, Pyrimidin, Pyridin, Pyrazol, Pyrrol, Tetrazol, Thiazol, Triazol, Thiophen. Thiadiazol, Benzimidazol, Benzofuran, Benzoxazol, Isochinolin, Chinolin. Bevorzugt sind 5- und 6gliedrige Heteroaromaten mit 1 bis 2 Stickstoff, Sauerstoff oder Schwefelatomen und insbesondere Furanyl und Thienyl. Als Substituenten der Heteroarylreste sind insbesondere NO₂, CN. Halogen, C₁₋₄-Alkyl und CF₃ geeignet.

Als gesättigte Heterocyclen NR¹⁴R¹⁵ seien beispielsweise Piperidin, Pyrrolidin, Morpholin, Thiomorpholin. Hexahydroazepin und Piperazin genannt. Der Heterocyclus kann 1 - 3fach substituiert sein mit C₁₋₄-Alkyl oder einem gegebenenfalls mit Halogen substituierten Phenyl-. Benzyl- oder Benzoylrest. Beispielsweise seien genannt: N-Methyl-piperazin. 2,6-Dimethylmorpholin. Phenylpiperazin und 4-(4-Fluorbenzoyl)-piperidin.

Bilden NR¹⁴R¹⁵ gemeinsam mit dem Stickstoffatom einen ungesättigten Heterocyclus, so seien beispielsweise Imidazol, Pyrrol, Pyrazol, Triazol, Benzimidazol und Indazol genannt, die ein- bis zweifach mit Phenyl, C₁₋₄-Alkyl, Halogen insbesondere Chlor oder CH₂-OH substituiert sein können.

Bedeutet R¹⁴ oder R¹⁵ Indanyl oder 1,2,3,4-Tetrahydronaphthyl, so kann dieser Rest jeweils in 1 - oder 2-Position verknüpft sein.

Bilden R⁴ und R⁵ gemeinsam mit zwei benachbarten Kohlenstoffatomen einen Carbocyclus, so kann dieser in beliebiger Position stehen und in beliebiger Position ein- oder zweifach mit NR¹⁴R¹⁵ substituiert sein. Bevorzugt ist einfache Subtsitution. Bevorzugt bedeuten R⁴ und R⁵ C₃-C₄-Alkylen,

R³ bedeutet C₁₋₅-Alkylen.

R⁵ bedeutet insbesondere Wasserstoff oder bildet gemeinsam mit R⁴ und mit zwei benachbarten Kohlenstoffatomen einen 5- oder 6gliedrigen Carbocyclus, der mit NR¹⁴R¹⁵ substituiert ist.

Bevorzugte Ausführungsformen für R⁶ sind Wasserstoff und Halogen und für R¹⁴ Wasserstoff und CO₂R²⁰.

Die Erfindung betrifft auch die Verwendung der erfindungsgemäßen Verbindungen zur Herstellung eines Arzneimittels zur Behandlung von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen.

Beispielsweise seien genannt:
Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korsakoffs Disease, Epilepsie, Erbrechen, Stress, Schlafstörungen, Schizophrenie, Depression, Migräne, Schmerz, Hypoglykämie, Demenz wie z.B. Alzheimersche Krankheit, HIV-Demenz und präsenile Demenz.

Ferner eignen sie sich zur Behandlung von Krankheiten des Herz-Kreislauf-Systems und zur Behandlung autoimmuner und/oder inflammatorischer Erkrankungen wie Hypotension, ARDS (adult respiratory distress syndrome), Sepsis oder Septischer Schock, Rheumatoider Arthritis. Osteoarthritis, von insulinabhängiger Diabetes Mellitus (IDDM), entzündlicher Erkrankung des Beckens /Darms (bowel disease), von Meningitis. Glomerulonephritis, akute und chronische Lebererkrankungen. Erkrankungen durch Abstoßung (beispielsweise allogene Herz-,Nieren- oder Lebertransplantationen) oder entzündlichen Hautkrankheiten wie Psoriasis und andere.

Auf Grund ihres Wirkprofils eignen sich die erfindungsgemäßen Verbindungen sehr gut zur inhibition der neuronalen NOS.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfsund/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch in Form von transdermalen Systemen und Sprays oder intrathekal erfolgen. Als Hilfsstoffe für die gewünschte Arzneimittelformulierung sind die dem Fachmann bekannten inerten organischen und anorganischen Trägermaterialien geeignet wie z.B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide. aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder. wie zum Beispiel Lactose, Mais- oder Kartoffelstärke, geeignet. Die Anwendung kann auch in flüssiger Form erfolgen. wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1 - 2000 mg, vorzugsweise 20 - 500 mg, wobei die Dosis als einmal zu verabreichende Einzeldosis oder unterteilt in zwei oder mehreren Tagesdosen gegeben werden kann.

Die NOS-inhibitorische Wirksamkeit der Verbindungen der Formel (I) und deren physiologisch verträglicher Salze kann nach den Methoden von Bredt und Snyder in Proc. Natl. Acad. Sci. USA (1989) **86**, 9030-9033. bestimmt werden. Die bNOS-Inhibition des Beispiels 8 (4-Amino-8-chlor-7-(3-chlorbenzylamino)-ethyl-2,3,3a,9b-tetrahydro-1H-cyclopenta[c]chinolin Dihydrochlorid) beträgt IC₅₀= 190nM.

Die Herstellung der erfindungsgemäßen Verbindungen erfolgt dadurch, daß man eine Verbindung der Formel (II) oder deren Salz 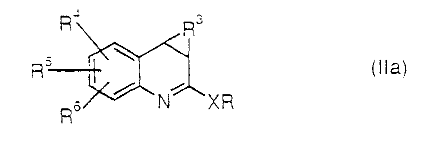 oder 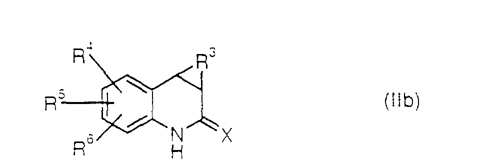 worin R³ bis R⁶ die obige Bedeutung haben. R Methyl oder Ethyl bedeutet und X = O oder S ist, mit Ammoniak, primären oder sekundären Aminen, Hydroxylamin und seinen Derivaten oder Hydrazin und seinen Derivaten umsetzt und gewünschtenfalls anschließend die isomeren trennt oder die Salze bildet.

Die Umsetzung mit Ammoniak gelingt unter Druck in Autoklaven bei Ammoniaküberschuß bei tiefen Temperaturen (-78 °C) oder durch Rühren in mit Ammoniak gesättigten Methanol. Bevorzugt werden Thiolactame umgesetzt. Wird mit Aminen umgesetzt, so stellt man aus dem Lactam oder Thiolactam zunächst den Iminoether oder Iminothioether ais Zwischenverbindung dar (z.B. mit Methyliodid oder Dimethylsulfat) und setzt diesen mit oder ohne Isolierung mit den entsprechenden Aminen oder deren Salzen um.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomeren können auch durch Chromatographie an chiralen Phasen sowie durch stereoselektive Synthese erhalten werden.

Die Herstellung der Salze erfolgt in üblicher Weise. indem man eine Lösung der Verbindung der Formel (I) mit der äquivalenten Menge oder einem Überschuß einer Säure. die gegebenenfalls in Lösung ist. versetzt und den Niederschlag abtrennt oder in üblicher Weise die Lösung aufarbeitet.

Soweit die Herstellung der Ausgangsverbindungen nicht beschrieben wird, sind diese bekannt und käuflich oder analog zu bekannten Verbindungen oder nach hier beschriebenen Verfahren herstellbar.

An den Vorstufen werden gewünschtenfalls Sulfide oxidiert. Ester verseift, Säuren verestert. Hydroxylgruppen verethert oder acyiiert. Amine acyliert, alkyliert, diazotiert. halogeniert, NO₂ eingeführt oder reduziert, mit Isocyanaten oder Isothiocyanaten umgesetzt, die Isomeren getrennt oder die Salze gebildet.

Zusätzlich kann durch elektrophile aromatische Substitution eine Nitrogruppe oder Halogen, insbesondere Chlor und Brom, eingeführt werden. Dabei entstehende Gemische können in üblicher Weise, auch mittels HPLC, getrennt werden. Wenn ein Nitril vorliegt, kann dieses nach bekannten Verfahren verseift werden oder in das entsprechende Amin, Tetrazol oder Amidoxim übergeführt werden.

Die Reduktion der Nitrogruppe oder gegebenenfalls der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat oder Ameisensäure in bekannter Weise benutzt werden. Reduktionsmittel wie Zinn(II)chlorid oder Titan(III)chlorid können ebenso verwendet werden wie komplexe Metallhydride. eventuell in Gegenwart von Schwermetallsalzen. Für Nitrogruppen bewährt hat sich die Reduktion mit Zink in Wasser-Ethanol-THF/Ammoniumchlorid oder Eisen in Essigsäure.

Wird eine einfache oder mehrfache Alkylierung einer Aminogruppe oder einer CH-aciden Kohlenstoffposition gewünscht, so kann nach üblichen Methoden beispielsweise mit Alkylhalogeniden alkyliert werden. Gegebenenfalls ist Schutz der Lactamgruppe ais Anion durch ein 2. Equivaient Base oder durch eine passende Schutzgruppe erforderlich.

Die Acylierung der Aminogruppe erfolgt in üblicher Weise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt.

Die Einführung einer NO₂-Gruppe gelingt durch eine Reihe von bekannten Nitrierungsmethoden. Beispielsweise kann mit Nitraten oder mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser, Essigsäure oder konz. Schwefelsäure als Lösungsmittel bei Temperaturen zwischen -10 °C und 30 °C.

Thiolactame der Formel (IIb, X = S) erhält man beispielsweise aus Lactamen mit Phosphorpentasulfid (P₄S₁₀) oder 2,4-Bis(4-methoxyphenyl)-1.3,2,4-dithiaphosphetan-2,4-disulfid (Lawessons Reagenz) in geeigneten Lösungsmitteln. Verbindungen der Formell (IIa) können beispielsweise durch Umsetzung mit Meerwein-Reagenz (Trimethyloxoniumtetrafluoroborat) erhalten werden.

Die Erfindung betrifft auch die Verbindungen der Formel IIb 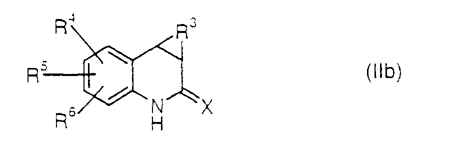 worin R³ bis R⁶ die obige Bedeutung haben und X = O oder S ist, die Zwischenverbindungen bei der Herstellung pharmakologisch wirksamer Verbindungen darstellen und nach den beschriebenen Verfahren erhalten und weiterverarbeitet werden.

Die Herstellung der Verbindungen der Formel (IIb, X = O) erfolgt nach der dem Fachmann bekannten Weise. Sie kann beispielsweise dadurch erfolgen. daß man eine Verbindung der Formel (III) 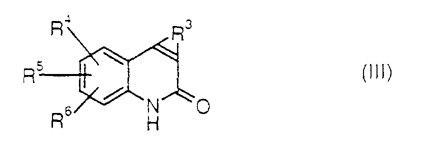 mit einem Alkali- oder Erdalkalimetal oder einem Amalgam derselben in Alkohol zu Lactam (II) reduziert (vgl. B.K. Blount, W.H. Perkin, S.G.P. Plant, *J. Chem. Soc.* **1929,** 1975; R. Brettle, S.M. Shibib, *J*.*Chem*. *Soc*. *Perkin Trans 1*, **1981**, 2912).

Die Herstellung der Chinolone vom Typ (III) erfolgt in der dem Fachmann bekannten Weise, z. B. nach B.K. Blount, W.H. Perkin, S.G.P. Plant, *J*. *Chem*. *Soc*. **1929,** 1975; W. Ried, W. Käppeler, *Liebigs Ann. Chem.* **1965,** *688*, 177; L.A. White, R.C. Storr, *Tetrahedron* **1996**, *52*, 3117.

Die Einführung der Substituenten R⁴-R⁶ kann auch auf der Stufe der Verbindung (III) erfolgen und findet wie oben beschrieben statt.

Beispielsweise kann die Herstellung der Verbindungen der Formel II mit R⁴ in der Bedeutung eines mit NR¹⁴R¹⁵ substituierten Alkylrestes durch reduktive Aminierung des entsprechenden Aldehyds bzw. wenn R⁴ und R⁵ einen 5- oder 6gliedrigen Carbocyclus bilden, der mit NR¹⁴R¹⁵ substituiert ist, durch reduktive Aminierung des entsprechenden Ketons erfolgen. Wird die Einführung eines Heteroaryirestes NR¹⁴R¹⁵ gewünscht. so kann das entsprechende Halogenderivat nucleophil substituiert werden. Ist eine primäre oder sekundäre Aminogruppe vorhanden, so kann es vorteilhaft sein, diese intermediär zu schützen, beispielsweise durch Einführung einer *tert*-Butoxycarbonylgruppe, die nach der Amidin-Bildung in üblicher Weise abgespalten wird.

Neue Verbindungen wurden durch eine oder mehrere der folgenden Methoden charakterisiert: Schmelzpunkt, Massenspektroskopie, Infrarotspektroskopie, Nuklear-magnetische Resonanzspektroskopie (NMR). NMR Spektren wurden mit einem Bruker 300 MHz Gerät gemessen, die (deuterierten) Lösemittel werden jeweils angegeben und wie folgt abgekürzt: CDCl₃ (Chloroform), CD₃OD ([D₄]-Methanol), DMSO ([D₆]-Dimethylsulfoxid). Verschiebungen sind in delta und ppm angegeben. Es bedeuten: m (Multiplett, mehrere Signale), s (Singulett), d (Dublett), dd (Doppeldublett usw.), t (Triplett), q (Quartett), H (Wasserstoffprotonen). Ferner bedeuten: THF (Tetrahydrofuran), DMF (N,N-Dimethylformamid), MeOH (Methanol), ml (Milliliter). Alle Lösemittel sind p.A.Qualität, wenn nicht anders vermerkt. Alle Reaktionen werden unter Schutzgas ausgeführt, es sei denn es handelt sich um wäßrige Lösungen. Schmelzpunkte werden in Grad Celsius angegeben und sind nicht korrigiert.

Nachfolgend wird die Darstellung einiger Vorstufen. Zwischenprodukte und Produkte exemplarisch beschrieben.

### Ausgangsverbindungen

### 7-Brom-1,2,3,5-tetrahydrocyclopenta[c]chinolin-4-on

Zu einer Lösung von 20.0 g (101 mmol) 3-Bromphenylisocyanat in 100 ml Chloroform werden vorsichtig 15.2 ml (101 mmol) 1-Morpholino-1-cyclopenten getropft. Der Ansatz wird 15 min unter Rückfluß erhitzt und i. Vak. eingeengt. Säulenchromatographie an Kieselgel mit Hexan - Essigester lieferte 25.0 g (88.6 mmol) Cyclopentan-2-on-1-carbonsäure-(3-bromphenyl)amid. Diese werden mit werden mit 83 ml konz. Schwefelsäure versetzt und 30 min bei 90 °C gerührt. Nach Abkühlen auf Raumtemp. wird der Ansatz auf 600 g Eis gegossen, der ausgefallene Feststoff abgesaugt und aus Ethanol umkristallisiert: 17.0 g Produkt.
¹H-NMR ([D₆]DMSO): 2.11 (pent., 2H), 2.74 (t. 2H). 3.07 (t, 2H), 7.34 (dd, 1H), 7.49 (d, 1H), 7.52 (d, 1 H), 11.68 (s, 1 H).

### 7-(2-Furanyl)-1,2,3,5-tetrahydrocyclopenta[c]chinolin-4-on

Eine Suspension von 1.32 g (5.0 mmol) 7-Brom-1,2,3,5-tetrahydrocyclopenta[c]chinoiin-4-on in 200 mL Toluol wird mit 1.7 ml (5.5 mmol) 2-(Tributylstannyl)furan und 0.29 g (0.25 mmol) Tetrakis(triphenylphosphin)palladium versetzt. Das Reaktionsgemisch wird entgast, mit Stickstoff belüftet, 15 h bei Raumtemp. gerührt und 4.5 h auf 110°C erhitzt. Der Ansatz wird mit Kieselgel versetzt und i. Vak. eingeengt. Säulenchromatographie des Rückstands an Kieselgel mit Hexan-Essigester liefert 1.33 g Produkt.
¹H-NMR ([D₆]DMSO/CDCl₃): 2.00 (pent., 2H), 2.70 (t, 2H). 2.91 (t, 2H), 6.30 (dd, 1 H), 6.58 (d. 1H), 7.22 - 7.31 (m, 3H), 7.44 (d. 1H), 11.09 (br.s, 1H).

### 7-(2-Furanyl)-1,2,3,3a,5,9b-hexahydrocyclopenta[c]chinolin-4-on

Eine Lösung von 1.32 g (5.3 mmol) 7-(2-Furanyl)-1,2,3,5-tetrahydrocyclopenta[c]chinolin-4-on in 300 ml Methanol wird mit 2.58 g (10.6 mmol) Magnesium und 0.06 ml Essigsäure versetzt. Nach 15 h bei Raumtemp. werden weitere 1.29 g (5.3 mmol) Magnesium dazugegeben. Der Ansatz wird 15 h bei Raumtemp. gerührt, mit 10 proz. Salzsäure (500 ml) behandelt und mit Essigester (3 x 300 ml) extrahiert. Die vereinigten Extrakte werden getrocknet (Na₂SO₄) und i. Vak. eingeengt. Säulenchromatographie an Kieselgel mit Hexan-Essigester liefert 0.49 g Produkt.
¹H-NMR (CDCl₃): 1.60 - 1.80 (m, 3H), 2.05 - 2.20 (m, 2H), 2.34 (m, 1 H), 2.98 (td, 1 H), 3.26 (q, 1 H), 6.48 (dd, 1 H), 6.64 (d, 1H), 7.04 (d, 1H), 7.22 (d, 1 H), 7.32 (dd. 1 H), 7.47 (d, 1H), 8.04 (br.s, 1H).

### 1,2,3,3a,5,9b-Hexahydrocyclopenta[c]chinolin-4-on-7-carponsäure

Eine Suspension von 0.37 g (1.5 mmol) 7-(2-Furanyl)-1,2,3,3a,5,9b-hexahydrocyclopenta[c]chinolin-4-on werden in 50 ml Acetonitril-Tetrachlorkohlenstoff-Wasser (2:1:2) suspendiert wird mit 4.81 g (22.5 mmol) Natriumperiodat und 40 mg (0.3 mmol) Ruthenium(IV)oxid versetzt. Nach 24 h bei Raumtemp. wird der Ansatz mit Wasser (100 ml) verdünnt und mit Essigester (3 x 100 ml) extrahiert. Die vereinigten Extrakte werden getrocknet (Na₂SO₄) und i. Vak. eingeengt. Der Rückstand wird in 100 ml 0.5 M Kaliumhydroxidlösung gelöst. Die Lösung wird mit Methyl-*tert*-butylether (2 x 100 ml) gewaschen, mit konz. Salzsäure angesäuert und mit Essigester (3 x 100 ml) extrahiert. Die vereinigten Extrakte werden getrocknet (Na₂SO₄) und i. Vak. eingeengt: 213 mg Produkt.
¹H-NMR ([D₆]DMSO/CDCl₃): 1.38 - 1.58 (m, 3H), 1.80 - 1.95 (m. 2H), 2.11 (m, 1H), 2.71 (td, 1H), 3.08 (q, 1H), 7.03 (d. 1H), 7.34 (d, 1 H), 7.42 (dd, 1 H), 9.35 (s. 1H).

### 7-Hydroxymethyl-1,2,3,3a,5,9b-hexahydrocyclopenta[c]chinolin-4-on

Eine Lösung von 150 mg (0.65 mmol) 1,2.3,3a,5,9b-Hexahydrocyclopenta [c]-chinolin-4-on-7-carbonsäure in 20 ml THF wird mit 0.10 ml (0.70 mmol) Triethylamin und 0.07 ml (0.70 mmol) Chlorameisensäure-ethylester bei Raumtemp. versetzt. Nach 10 min werden 76 mg (2.0 mmol) Natriumborhydrid hinzugefügt und innerhalb von 20 min 10 ml Methanol dazugetropft. Der Ansatz wird 15 h bei Raumtemp. gerührt, mit Essigester (100 ml) verdünnt, mit 20 proz. Citronensäure (50 m1), und ges. NaCl (50 ml) gewaschen, getrocknet (Na₂SO₄) und i. Vak. eingeengt. Säuienchromatographie an Kieseigel mit Hexan-Essigester liefert 65 mg Produkt.
¹H-NMR (CDCl₃): 1.54 - 1.79 (m, 3H), 2.02 - 2.18 (m, 3H), 2.30 (m, 1H), 2.93 (td, 1 H), 3.24 (q, 1 H), 4.67 (d, 2H), 6.80 (s, 1H), 6.99 (d, 1 H), 7.18 (d, 1 H), 8.26 (s, 1H).

### 1,2,3,3a,5,9b-Hexahydrocyclopenta[c]chinolin-4-on-7-carbaldehyd

Eine Lösung von 187 mg (0.86 mmol) 7-Hydroxymethyl-1,2,3,3a,5,9bhexahydrocyclopenta[c]chinolin-4-on in 20 ml Chloroform-Dichlormethan-Acetonitril (2:1:1) wird mit 151 mg (1.29 mmol) N-Methylmorpholin-N-oxid und 1.5 g Molekularsieb 4 Å versetzt. Nach Zugabe von 15 mg (0.043 mmol) Tetrapropylammoniumperruthenat (TPAP) wird der Ansatz 2.5 h bei Raumtemp. gerührt. bevor weitere 10 mg (0.028 mmol) TPAP hinzugefügt werden. Nach 1.5 h wird Kieselgel zum Reaktionsgemisch gegeben un das Lösungsmittel i. Vak. abdestilliert. Säulenchromatographie mit Hexan-Essigester liefert 156 mg Produkt.
¹H-NMR (CDCl₃): 1.60 - 1.83 (m, 3H), 2.08 - 2.23 (m, 2H), 2.40 (m, 1H), 3.01 (td, 1 H), 3.34 (q, 1 H), 7.30 (d, 1H), 7.49 (d. 1 H), 7.53 (dd, 1 H), 8.63 (br.s, 1 H), 9.96 (s, 1H).

### 7-(3-Chlorbenzylamino)methyl-1,2,3,3a,5,9b-hexahydrocyclopenta[c]chinolin-4-on

Eine Lösung von 150 mg (0.70 mmol) 1,2,3,3a,5,9b-Hexahydrocyclopenta[c]-chinolin-4-on-7-carbaldehyd in 15 ml 1,2-Dichlorethan wird mit 0.10 ml (0.84 mmol) und 267 mg (1.26 mmol) Natrium(triacetoxy)borhydrid versetzt. Nach Zugabe von 0.04 ml Essigsäure wird der Ansatz 15 h bei Raumtemp. gerührt, mit Essigester (100 ml) verdünnt, Wasser (20 ml ) gewaschen, getrocknet (Na₂SO₄) und i. Vak. eingeengt. Säulenchromatographie an Kieselgel mit Dichlormethan-Ethanol liefert 214 mg Produkt.
¹H-NMR (CDCl₃): 1.58 - 1.78 (m, 3H), 2.03 - 2.19 (m, 2H), 2.31 (m, 1H), 2.95 (td, 1 H), 3.26 (q, 1 H), 3.75 (s, 2H), 3.80 (s, 2H), 6.75 (dd, 1H), 6.97 (dd, 1H), 7.16 (d, 1 H), 7.22 - 7.32 (m. 3H), 7.37 (s, 1H), 8.41 (br.s, 1 H).

### 7-(N-tert-Butoxycarbonyl-3-chlorbenzylamino)methyl-1,2,3,3a,5,9bhexahydrocyclopenta[c]chinolin-4-on

Eine Lösung von 207 mg (0.61 mmol) 7-(3-Chlorbenzylamino)methyl-1,2,3,3a,5,9b-hexahydrocyclopenta[c]chinolin-4-on in 20 ml THF wird mit 201 mg (0.92 mmol) Di-*tert*-butylkohlensäureanhydrid und 4 mg (0.03 mmol) 4-(Dimethylamino)pyridin versetzt und 3 h bei Raumtemp. gerührt, bevor weitere 201 mg (0.92 mmol) Di-*tert*-butylkohlensäureanhydrid dazugegeben werden. Nach 15 h bei Raumtemp. wird der Ansatz mit Essigester (100 ml) verdünnt, mit 20 proz. Citronensäure (50 ml) und ges. NaCl (20 ml) gewaschen, getrocknet (Na₂SO₄) und i. Vak. eingeengt. Säulenchromatographie an Kieselgel mit Hexan-Essigester liefert 76 mg Produkt.
¹H-NMR (CDCl₃): 1.52 (s, 9H), 1.40 - 1.80 (m, 3H), 2.00 - 2.20 (m, 2H), 2.34 (m, 1 H), 2.95 (td, 1 H), 3.26 (q, 1 H), 4.32 (br., 2H), 4.40 (br., 2H), 6.55 (br., 1 H), 6.85 (br., 1 H), 7.08 (br., 1H), 7.13 (d, 1 H), 7.21- 7.32 (m, 3H), 7.56 - 7.70 (br., 1 H).
MS (FAB) m/e = 441 (M⁺)
Darüberhinaus werden 104 mg 5- *tert*-Butoxycarbonyl-7-(*N*-*tert*-butoxycarbonyl-3-chlor benzylamino)methyl-1,2,3,3a,5,9b-hexahydrocyclopenta[c]chinolin-4-on isoliert.

### 7-(N-tert-Butoxycarbonyl-3-chlorbenzylamino)methyl-1,2,3,3a,5,9bhexahydrocyclopenta[c]chinolin-4-thion

Eine Lösung von 75 mg (0.17 mmol) 7-(*N*-*tert*-Butoxycarbonyl-3-chlorbenzylamino)methyl-1,2,3,3a,5,9b-hexahydrocyclopenta[c]chinolin-4-on in 5 ml 1,2-Dimethoxyethan wird mit 138 mg (0.34 mmol) Lawessons Reagenz versetzt. Nach 1.5 h bei Raumtemp. wird der Ansatz 0.75 h unter Rückfluß erhitzt und anschließend i. Vak. eingeengt. Säulenchromatographie an Kieselgel mit Hexan-Essigester liefert 65 mg Produkt.
¹H-NMR (CDCl₃): 1.53 (s, 9H), 1.48 - 1.78 (m, 2H), 1.93 (m, 1 H), 2.10 - 2.39 (m, 2H), 3.23 - 3.36 (m, 2H), 4.33 (br., 2H), 4.49 (br., 2H), 6.58 und 6.68 (br., 1H), 6.95 (br.d, 1H), 7.08 (br., 1H), 7.17 (br.s, 1H), 7.21 (d, 1 H), 7.24 - 7.31 (m, 2H), 9.40 (s, 1 H).
MS (FAB) m/e = 457 (M⁺)

### 7-Vinyl-1,2,3,5-tetrahydrocyclopenta[c]chinolin-4-on

Eine Lösung von 2.0 g (7.6 mmol) 7-Brom-1,2,3,5-tetrahydrocyclopenta[c]-chinolin-4-on, 2.6 mL (9.9 mmol) Vinyltributylzinn und 0.44 g (0.38 mmol) Tetrakis(triphenylphosphin)palladium wird entgast und mit Stickstoff belüftet. Nach sechsstündigem Erwärmen auf 110 °C wird der Ansatz eingeengt und der Rückstand auf Kieselgel aufgezogen. Säulenchromatographie an Kieselgel mit Hexan-Essigester liefert 1.41 g Produkt.
¹H-NMR (CDCl₃): 2.26 (pent., 2H), 3.05 (t, 2H), 3.16 (t, 2H), 5.41 (d. 1H), 5.91 (d, 1H), 6.82 (dd, 1H), 7.33 (d, 1 H), 7.38 (s, 1 H), 7.49 (d, 1 H), 11.22 (br.s, 1H).

### 7-Oxiranyl-1,2,3,5-tetrahydrocyclopenta[c]chinolin-4-on

Eine Lösung von 1.41 g (6.7 mmol) 7-Vinyl-1,2,3,5-tetrahydrocyclopenta[c]-chinolin-4-on in 200 ml Chloroform wird bei Raumtemp. mit *m*CPBA versetzt. Nach 15 h bei Raumtemp. wird der Ansatz mit ges. Na₂SO₃ (2 x 100 ml) gewaschen, getrocknet (Na₂SO₄) und i. Vak. eingeengt. Säulenchromatographie an Kieselgel mit Hexan-Essigester liefert 0.59 g Produkt.
¹H-NMR (CDCl₃): 2.25 (pent., 2H), 2.85 (dd, 1H), 3.03 (t, 2H), 3.13 (t. 2H), 3.20 (dd, 1 H), 3.98 (dd, 1H), 7.11 (dd, 1H), 7.35 (d, 1H), 7.49 (d, 1 H), 11.44 (br.s. 1 H).

### 7-Hydroxyethyl-1,2,3,3a,5,9b-hexahydrocyclopenta[c]chinolin-4-on

Eine Lösung von 0.59 g (2.6 mmol) 7- Oxiranyl-1,2,3,5-tetrahydrocyclopenta[c]-chinolin-4-on in 100 ml Methanol wird mit 1.26 g (52.0 mmol) Magnesium und 0.06 ml Essigsäure versetzt. Der Ansatz wird 4 h bei Raumtemp. gerührt und mit weiteren 0.63 g (26.0 mmol) Magnesium versetzt. Nach 15 h bei Raumtemp. wird das Reaktionsgemisch mit 200 ml 10 proz. Salzsäure angesäuert und mit Essigester (3 x 200 ml) extrahiert. Die vereinigten Extrakte werden getrocknet (Na₂SO₄) und i. Vak. eingeengt. Säulenchromatographie an Kieselgel mit Hexan-Essigester liefert 0.39 g Produkt.
¹H-NMR (CDCl₃): 1.57 - 1.78 (m, 3H), 2.01 - 2.18 (m, 2H), 2.29 (m, 1H), 2.82 (t, 2H), 2.93 (td. 1 H), 3.23 (q, 1 H), 3.86 (t, 2H), 6.62 (d, 1H), 6.88 (dd, 1 H), 7.15 (d, 1 H), 8.31 (br.s, 1H).

### 7-(3-Chlorbenzylamino)ethyl-1,2,3,3a,5,9b-hexahydrocyclopenta[c]chinolin-4-on

Eine Lösung von 0.19 g (0.82 mmol) 7-Hydroxyethyl-1,2,3,3a,5,9b-hexahydrocyclopenta[c]chinolin-4-on in 20 ml Dichlormethan wird bei 0 °C mit 0.38 g (0.90 mmol) 1,1,1-Triacetoxy-1,1-dihydro-1,2-benziodoxol-3(1H)-on (D.B. Dess. J.C. Martin, *J. Am. Chem. Soc.* **1991**, *113*, 7277) versetzt. Der Ansatz wird 10 min bei 0 °C und 40 min bei Raumtemp. gerührt, mit Dichlormethan (100 ml) verdünnt, mit ges. NaHCO₃ (30 ml) gewaschen, getrocknet (Na₂SO₄) und i. Vak. eingeengt. Der Rückstand wird in 1,2-Dichlorethan (20 ml) gelöst und mit 0.11 ml (0.90 mmol) 3-Chlorbenzylamin, 0.30 g (1.40 mmol) Natrium-(triacetoxy)borhydrid und 0.05 ml (0.85 mmol) Essigsäure versetzt. Nach 20 h bei Raumtemp. wird der Ansatz mit Essigester (150 ml) verdünnt, mit Wasser (2 x 50 ml) gewaschen, getrocknet (Na₂SO₄) und i. Vak. eingeengt. Säulenchromatographie an Kieselgel mit Dichlormethan-Ethanol liefert 69 mg Produkt.
¹H-NMR (CDCl₃): 1.54 - 1.77 (m, 3H), 2.01 - 2.17 (m, 2H), 2.27 (m, 1 H), 2.82 (t, 2H), 2.90 (td, 1 H), 2.94 (t, 2H), 3.20 (q, 1 H), 3.87 (s, 2H), 5.13 (br.), 6.65 (d, 1 H), 6.80 (dd, 1 H), 7.19 (d, 1 H), 7.22 (m. 3H), 7.35 (d, 1H), 8.80 (br.s, 1 H).

### 7-(N-tert-Butoxycarbonyl-3-chlorbenzylamino)ethyl-1,2,3,3a,5,9bhexahydrocyclopenta[c]chinolin-4-thion

Eine Lösung von 69 mg (0.19 mmol) 7-(3-Chlorbenzylamino)ethyl-1,2,3,3a,5,9bhexahydrocyclopenta[c]chinolin-4-on in 10 ml Dichlormethan wird mit 46 mg (0.21 mmol) Di-*tert*-butylkohlensäureanhydrid und 2 mg (0.02 mmol) 4-(Dimethylamino)pyridin versetzt. Nach 15 h bei Raumtemp. wird der Ansatz mit Methyl-*tert*-butylether (100 ml) verdünnt, mit 10 proz. Citronensäure und ges.

NaCl gewaschen, getrocknet (Na₂SO₄) und i. Vak. eingeengt. Der Rückstand wird in 1,2-Dimethoxyethan (10 ml) gelöst und mit 202 mg (0.5 mmol) Lawessons Reagenz behandelt. Nach 6 h bei Raumtemp. wird der Ansatz eingeengt und der Rückstand säulenchromatograpisch mit Hexan-Essigester gereinigt: 48 mg Produkt.
¹H-NMR (CDCl₃): 1.45 (s, 9H), 1.54 - 1.75 (m, 2H), 1.89 (m, 1 H), 2.07 - 2.36 (m, 3H), 2.55 (br., 2H), 3.21 - 3.48 (m, 4H), 4.35 (br., 2H), 6.52 (br., 1 H), 6.88 (br., 1 H), 7.09 (br., 1H), 7.15 (d, 1 H), 7.18 (m, 1 H), 7.25 (m, 2H), 9.32 (s, 1 H).

### Beispiel 1

### 4-Amino-7-(N-tert-butoxycarbonyl-3-chlorbenzylamino)methyl-2,3,3a,9btetrahydro-1H-cyclopenta[c]chinolin

60 mg (0.13 mmol) 7-(*N*-*tert*-Butoxycarbonyl-3-chlorbenzylamino)methyl-1,2,3,3a,5,9b-hexahydrocyclopenta[c]chinolin-4-thion werden in 10 ml 7 M ammoniakalischem Methanol suspendiert. Nach 15 h bei Raumtemp. wird die Reaktionslösung eingeengt und der Rückstand säulenchromatographisch an Kieselgel mit Dichlormethan-Ethanol gereinigt: 47 mg Produkt.
¹H-NMR (CDCl₃): 1.50 (s, 9H), 1.63 - 2.00 (m, 4H), 2.16 (m, 2H), 2.77 (q, 1 H), 3.29 (q, 1 H), 4.29 (br., 2H), 4.38 (br., 2H), 4.73 (br. 2H), 6.85 (br., 2H), 7.09 (d, 1H), 7.01 - 7.30 (m, 4H).
MS (FAB) m/e = 440 (M⁺)

### Beispiel 2

### 4-Amino-7-(3-chlorbenzylamino)methyl-2,3,3a,9b-tetrahydro-1H-cyclopenta[c]-chinolin Dihydrochlorid

44 mg (0.10 mmol) 4-Amino-7-(*N*-*tert*-butoxycarbonyl-3-chlorbenzylamino)-methyl-2,3,3a,9b-tetrahydro-1H-cyclopenta[c]chinolin wird in 3 ml 4 M salzsaurem Dioxan 2 h bei Raumtemp. gerührt. Nach Zugabe von 1 ml Toluol wird die Lösung auf 1 ml eingeengt und dekantiert. Der Rückstand wird in 3 ml Methanol gelöst und die Lösung eingeengt. Der Rückstand wird mit 1 ml Chloroform behandelt und das Lösungsmittel abdestilliert: 40 mg.
¹H-NMR (CD₃OD): 1.53 - 1.79 (m, 3H), 2.06 (m, 1 H), 2.17 - 2.33 (m , 2H), 3.19 (q, 1 H), 3.49 (m, 1H), 4.18 (s, 2H), 4.21 (s, 2H), 7.22 (s, 1 H), 7.28 (d, 1 H), 7.38 (s, 3H), 7.40 (d, 1 H), 7.50 (s, 1 H).
MS (FAB) m/e = 340 ([M - 2 HCI]⁺)

### Beispiel 3

### 4-Amino-7-(N-tert-butoxycarbonyl-3-chlorbenzylamino)ethyl-2,3,3a,9b-tetrahydro-1H-cyclopenta[c]chinolin

45 mg (0.096 mmol) 7-(*N*-*tert*-Butoxycarbonyl-3-chlorbenzylamino)ethyl-1,2,3,3a,5,9b-hexahydrocyclopenta[c]chinolin-4-thion werden in 20 ml 6 M ammoniakalischem Methanol gelöst. Nach 15 h bei Raumtemp. wird der Ansatz eingeengt und der Rückstand säulenchromatographisch mit Dichlormethan-Ethanol-33 proz. NH₄OH gereinigt; 25 mg Produkt.
¹H-NMR (CDCl₃): 1.38 - 1.55 (br., 9H), 1.60 - 1.95 (m, 4H), 2.07 - 2.22 (m, 2H), 2.66 - 2.82 (m, 3H), 3.22 - 3.48 (m, 3H), 4.03 (br., 2H), 4.25 - 4.40 (br., 2H), 6.70 - 6.85 (br., 2H), 7.04 (d. 1 H). 7.08 (m. 1 H), 7.15 - 7.24 (m, 3H).
MS (FAB) m/e = 454 (M⁺)

### Beispiel 4

### 4-Amino-7-(3-chlorbenzylamino)ethyl-2,3,3a,9b-tetrahydro-1H-cyclopenta[c]-chinolin Dihydrochlorid

19 mg (0.042 mmol) 4-Amino-7-(*N*-*tert*-butoxycarbonyl-3-chlorbenzylamino)-ethyl-2,3,3a,9b-tetrahydro-1H-cyclopenta[c]chinolin werden in 3 ml 4 M salzsaurem Dioxan gelöst. Nach 15 h bei Raumtemp. wird die Reaktionslösung auf 1 ml eingeengt und das Lösungsmittel dekantiert. Der Rückstand wird in Chloroform suspendiert und eingeengt: 17 mg glasartiger Feststoff.
¹H-NMR (CD₃OD): 1.60 - 1.88 (m, 3H), 2.10 - 2.42 (m, 3H), 3.06 (t, 2H), 3.24 (q, 1H), 3.31 (t, 2H), 3.53 (m, 1 H), 4.28 (s, 2H), 7.08 (s. 1 H), 7.17 (d, 1 H), 7.38 (d, 1 H), 7.48 (s, 3H), 7.61 (s, 1 H).
MS (EI) m/e = 353 ([M - 2 HCI]⁺)

### Beispiel 5

### 4-Amino-7-[1-(N-tert-butoxycarbonyl-3-chlorbenzylamino)propyl]-2,3,3a,9btetrahydro-1H-cyclopenta[c]chinolin

### Ausgangsverbindungen

### 7-(Methoxycarbonylethenyl)-1,2,3,5-tetrahydrocyclopenta[c]chinolin-4-on

Eine Suspension von 528 mg (2.0 mmol) 7-Brom-1,2,3,5-tetrahydrocyclopenta[c]chinolin-4-on, 0.36 ml (4.0 mmol) Acrylsäure-methylester, 116 mg (0.1 mmol) Tetrakis(triphenylphosphin)palladium und 0.56 ml (4.0 mmol) Triethylamin in 25 ml DMF wird 3 h bei 120 °C gerührt. Der Ansatz wird mit Essigester verdünnt. mit Wasser gewaschen, getrocknet (Na₂SO₄) und i. Vak. eingeengt. Reinigung des Rückstands an Kieselgel mit Dichlormethan-Ethanol liefert 550 mg Produkt.
¹H-NMR ([D₆]-DMSO): δ = 2.12 (pent, 2H), 2.80 (t, 2H), 3.12 (t, 2H), 3.77 (s, 3H), 6.61 (d, 1H), 7.52 (s. 1H), 7.56 (s, 2H), 7.67 (d, 1 H), 11.19 (br.s, 1 H).

### 7-(Methoxycarbonylethyl)-1,2,3,3a,5,9b-hexahydrocyclopenta[c]chinolin-4-on

Eine Lösung von 550 mg (2.0 mmol) 7-(Methoxycarbonylethenyl)-1,2,3,5-tetrahydrocyclopenta[c]chinolin-4-on in 130 ml Methanol-THF 3:1 wird mit 972 mg (40.0 mmol) Magnesium versetzt und 24 h bei Raumtemp. gerührt. Das Reaktionsgemisch wird über Glasfaser filtriert, der Filterrückstand mit Dichlormethan-Methanol gewaschen und die vereinigten Filtrate i. Vak. eingeengt. Reinigung des Rückstands an Kieselgel liefert 120 mg Produkt.
¹H-NMR (CDCl₃): δ = 1.57 - 1.77 (m, 3H), 2.02 - 2.18 (m, 2H), 2.31 (m, 1 H), 2.63 (t, 2H), 2.91 (t, 2H), 2.94 (td, 1 H), 3.23 (q, 1 H), 3.69 (s, 3H), 6.58 (d, 1 H), 6.84 (dd, 1 H), 7.12 (d, 1 H), 8.11 (br.s, 1 H).

### 7-[1-(3-Chlorbenzylamino)propyl]-1,2,3,3a,5,9b-hexahydrocyclopenta[c]chinolin-4-on

Zu einer Lösung von 510 mg (1.9 mmol) 7-(Methoxycarbonylethyl)-1,2,3,3a,5,9b-hexahydrocyclopenta[c]chinolin-4-on in 50 ml Toluol werden bei -70 °C 1.6 ml (1.9 mmol) 1.2 M Diisobutylaluminiumhydrid (DIBAH) in Toluol getropft. Nach 2 h bei -70 °C wird der Ansatz mit 0.75 ml (0.9 mmol) DIBAH-Lösung versetzt, 15 min gerührt, mit 3 ml Isopropanol und 1 ml Wasser behandelt und 2 h bei Raumtemp. gerührt. Die Reaktionslösung wird filtriert und i. Vak. eingeengt. Der Rückstand wird in 50 ml 1,2-Dichlorethan gelöst, mit 0.38 ml (3.1 mmol) 3-Chlorbenzylamin, 0.91 g (4.3 mmol) Natrium(triacetoxy)-borhydrid und 0.017 ml (0.29 mmol) Essigsäure versetzt und 24 h bei Raumtemp. gerührt. Der Ansatz wird mit Essigester verdünnt, mit Wasser gewaschen, getrocknet (Na₂SO₄) u. i. Vak. eingeengt. Säulenchromatographie an Kieselgel mit Essigester-Methanol liefert 290 mg Produkt.
MS (Cl) m/e = 369 (M⁺)

### 7-[1-(N-tert-Butoxycarbonyl-3-chlorbenzylamino)propyl]-1,2,3,3a,5,9bhexahydrocyclopenta[c]chinolin-4-on

Eine Lösung von 290 mg (0.79 mmol) 7-[1-(3-Chlorbenzylamino)propyl]-1,2,3,3a,5,9b-hexahydrocyclopenta[c]chinolin-4-on in 20 ml Dichlormethan wird mit 206 mg (0.94 mmol) Di-*tert*-butylkohlensäureanhydrid versetzt und 24 h bei Raumtemp. gerührt. Der Ansatz wird mit Dichlormethan verdünnt, mit Wasser gewaschen, getrocknet (Na₂SO₄) und i. Vak. eingeengt. Säulenchromatographie an Kieselgel mit Hexan-Essigester liefert 260 mg Produkt.
MS (FAB) m/e = 469 (M⁺)

### 7-[1-(N-tert-Butoxycarbonyl-3-chlorbenzylamino)propyl]-1,2,3,3a,5,9bhexahydrocyclopenta[c]chinolin-4-thion

Eine Lösung von 260 mg (0.55 mmol) 7-[1-( *N*-*tert*-Butoxycarbonyl-3-chlorbenzylamino)propyl]-1,2,3,3a,5,9b-hexahydrocyclopenta[c]chinolin-4-on und 597 mg (1.48 mmol) Lawessons Reagenz in 30 ml THF wird 1 h unter Rückfluß erhitzt. Nach Einengen i. Vak. wird der Rückstand säulenchromatograpisch mit Hexan-Essigester gereinigt: 230 mg Produkt.
MS (FAB) m/e = 485 (M⁺)

### 4-Amino-7-[1-(N-tert-butoxycarbonyl-3-chlorbenzylamino)propyl]-2,3,3a,9btetrahydro-1H-cyclopenta[c]chinolin

230 mg (0.47 mmol) 7-[1-(*N*-*tert*-Butoxycarbonyl-3-chlorbenzylamino)propyl]-1,2,3,3a,5,9b-hexahydrocyclopenta[c]chinolin-4-thion werden in 20 ml 7 M ammoniakalischem Methanol 24 h bei Raumtemp. gerührt. Nach Abdestillieren des flüchtigen Bestandteile i. Vak. wird der Rückstand säulenchromatographisch mit Dichlormethan-Methanol an Kieselgel gereinigt: 150 mg Produkt.
MS (FAB) m/e = 468 (M⁺)

### Beispiel 6

### 4-Amino-7-[1-(3-chlorbenzylamino)propyl]-2,3,3a,9b-tetrahydro-1Hcyclopenta[c]chinolin

150 mg (0.32 mmol) 4-Amino-7-[1-(*N*-*tert*-butoxycarbonyl-3-chlorbenzylamino)-propyl]-2,3,3a,9b-tetrahydro-1H-cyclopenta[c]chinolin werden in 5 ml 4 M salzsaurem Dioxan 30 min bei Raumtemp. gerührt. Die flüchtigen Bestandteile werden i. Vak. entfernt: 140 mg.
¹H-NMR (D₆-DMSO): 1.53 (m, 3H), 1.67 (m, 2H), 1.91 - 2.05 (m, 3H), 2.12 - 2.25 (m , 2H), 2.64 (t, 2H), 2.90 (m, 2H), 3.20 (q, 1 H), 3.43 (m, 1 H), 4.15 (t, 2H), 6.94 (d, 1 H), 7.03 (dd, 1 H), 7.28 (d, 2H), 7.43 - 7.55 (m, 3H), 7.68 (s, 1 H), 8.93 (s. 1 H), 9.29 (br., 2H), 9.70 (s, 1 H).
MS (FAB) m/e = 367 ([M - 2 HCl]⁺)

### Beispiel 7

### 4-Amino-7-(N-tert-butoxycarbonyl-3-chlorbenzylamino)ethyl-8-chlor-2,3,3a,9btetrahydro-1H-cyclopenta[c]chinolin

### Ausgangsverbindungen

### 7-Acetoxyethyl-1,2,3,3a,5,9b-hexahydrocyclopenta[c]chinolin-4-on

Eine Lösung von 560 mg (2.4 mmol) 7-Hydroxyethyl-1,2,3,3a,5,9b-hexahydrocyclopenta[c]chinolin-4-on in 10 ml Pyridin wird mit 5 ml Acetanhydrid 24 h bei Raumtemp. gerührt. Der Ansatz wird i. Vak. eingeengt und säulenchromatographisch an Kielsegel mit Hexan-Essigester gereinigt: 500 mg Produkt.
¹H-NMR (CDCl₃): 1.59 - 1.81 (m, 3H), 2.02 - 2.19 (m, 2H), 2.07 (s, 3H), 2.32 (m, 1 H), 2.91 (t, 2H), 2.96 (td, 1 H), 3.25 (q, 1 H), 4.28 (t, 2H), 6.62 (d, 1 H), 6.87 (dd, 1H), 7.15(d, 1H), 8.25 (br.s, 1H).

### 7-Acetoxyethyl-8-chlor-1,2,3,3a,5,9b-hexahydrocyclopenta[c]chinolin-4-on

Eine Lösung von 400 mg (1.5 mmol) 7-Acetoxyethyl-1,2,3,3a,5,9b-hexahydrocyclopenta[c]chinolin-4-on und 193 mg (1.5 mmol) *N*-Chlorsuccinimid in 40 ml DMF wird 6 Tage auf 100 °C erhitzt. Der Ansatz wird auf Eiswasser gegossen und mit Essigester extrahiert. Die org. Phase wird mit 10 proz. Schwefelsäure und Wasser gewaschen, getrocknet (Na₂SO₄) und i. Vak. eingeengt.

Säulenchromatographle an Kieselgel mit Hexan-Essigester liefert 380 mg Produkt.
¹H-NMR (CDCl₃): 1.59 - 1.78 (m, 3H), 2.02 - 2.20 (m, 2H). 2.06 (s. 3H), 2.30 (m, 1 H), 2.93 (td. 1H), 3.03 (t, 2H), 3.23 (q. 1 H), 4.29 (t, 2H), 6.67 (s, 1H), 7.20 (s, 1H), 8.51 (br.s. 1 H).

### 8-Chlor-7-hydroxyethyl-1,2,3,3a,5,9b-hexahydrocyclopenta[c]chinolin-4-on

380 mg (1.2 mmol) 7-Acetoxyethyl-8-chlor-1,2,3,3a,5,9b-hexahydrocyclopenta[c]chinolin-4-on wird in 30 ml Methanol gelöst und mit 341 mg (2.5 mmol) Kaliumcarbonat versetzt. Nach 3 Tagen bei Raumtemp. wird der Ansatz mit Essigester verdünnt, mit Wasser gewaschen, getrocknet (Na₂SO₄) und i. Vak. eingeengt. Säulenchromatographie an Kieselgel mit Essigester-Ethanol liefert 270 mg Produkt.
¹H-NMR (D₅-DMSO/CDCl₃): 1.38 - 1.63 (m, 3H), 1.85 (m. 2H), 2.14 (m, 1 H), 2.72 (td, 1H), 2.78 (t. 2H), 3.07 (q, 1 H), 3.60 (q, 2H), 4.14 (t, 1H), 6.72 (s, 1 H), 7.02 (s, 1H), 9.73 (s. 1H).

### 8-Chlor-7-(3-chlorbenzylamino)ethyl-1,2,3,3a,5,9b-hexanydrocyclopenta[c]chinolin-4-on

Zu einer Lösung von 0.18 ml (2.04 mmol) Oxalylchlorid in 4 ml 1,2-Dichlorethan werden bei -70 °C 0.22 ml (3.12 mmol) DMSO in 2 ml 1,2-Dichlorethan getropft. Nach 10 min bei -70 °C wird eine Lösung von 270 mg (1.02 mmol) 8-Chlor-7-hydroxyethyl-1,2,3,3a,5,9b-hexahydrocyclopenta[c]chinolin-4-on in 5 ml 1,2-Dichlorethan und 2 ml DMSO dazugetropft. Nach 2 h bei -70 °C wird der Ansatz mit 30 ml Dichlormethan verdünnt und 3 h bei -70 °C gerührt. Nach Zugabe von 1.27 ml (9.18 mmol) Triethylamin wird die Reaktionslösung 1 h bei Raumtemp. gerührt und i. Vak. eingeengt. Der Rückstand wird in 20 ml 1,2-Dichlorethan und 20 ml THF aufgenommen und mit 0.14 ml (1.53 mmol) 3-Chlorbenzylamin, 323 mg (1.53 mmol) Natrium(triacetoxy)borhydrid und 0.6 ml (10.2 mmol) Essigsäure versetzt. Nach 24 h bei Raumtemp. wird der Ansatz mit Essigester verdünnt, mit Wasser gewaschen, getrocknet (Na₂SO₄) und i. Vak. eingeengt. Säulenchromatographie an Kieselgel mit Dichlormethan-Methanol liefert 220 mg Produkt.
¹H-NMR (D₆-DMSO/CDCl₃): 1.33 - 1.66 (m, 3H), 1.99 (m, 2H). 2.08 (m, 1 H), 2.76 (t, 2H), 2.96 (br., 2H), 3.12 (q, 1 H), 4.25 - 4.36 (m, 4H), 5.72 (br., 1 H), 7.07 - 7.23 (m, 5H), 7.16 (s, 1H), 7.40 (s, 1H), 9.89 (s, 1 H).

### 7-(N-tert-Butoxycarbonyl-3-chlorbenzylamino)ethyl-8-chlor-1,2,3,3a,5,9bhexahydrocyclopenta[c]chinolin-4-on

Eine Lösung von 220 mg (0.57 mmol) 8-Chlor-7-(3-chlorbenzylamino)ethyl-1,2,3,3a,5,9b-hexahydrocyclopenta[c]chinolin-4-on in 20 ml Dichlormethan wird mit 149 mg (0.68 mmol) Di-*tert*-butylkohlensäureanhydrid versetzt. Nach 24 h bei Raumtemp. wird der Ansatz mit Dichlormethan verdünnt, mit Wasser gewaschen, getrocknet (Na₂SO₄) und i. Vak. eingeengt. Säulenchromatographie an Kieselgel mit Hexan-Essigester liefert 90 mg Produkt.
MS (Cl) m/e = 489.

### 7-(N-tert-Butoxycarbonyl-3-chlorbenzylamino)ethyl-8-chlor-1,2,3,3a,5,9bhexahydrocyclopenta[c]chinolin-4-thion

Eine Lösung von 90 mg (0.18 mmol) 7-(*N*-*tert*-Butoxycarbonyl-3-chlorbenzylamino)ethyl-8-chlor-1,2,3,3a,5,9b-hexahydrocyclopenta[c]chinolin-4-on und 194 mg (0.48 mmol) Lawessons Reagenz werden 1 h bei Raurntemp, gerührt und 1 h unter Rückfluß erhitzt. Nach einengen i. Vak. wird der Rückstand säulenchromatographisch mit Hexan-Essigester gereinigt: 80 mg Produkt.
MS (CI) m/e = 505.

### 4-Amino-7-(N-tert-butoxycarbonyl-3-chlorbenzylamino)ethyl-8-chlor-2,3,3a,9btetrahydro-1H-cyclopenta[c]chinolin

80 mg (0.16 mmol) 7-(*N*-*tert*-Butoxycarbonyl-3-chlorbenzylamino)ethyl-8-chlor-1,2,3,3a,5,9b-hexahydrocyclopenta[c]chinolin-4-thion werden in 20 ml 7 M ammoniakalischem Methanol 24 h bei Raumtemp. gerührt. Der Ansatz wird eingeengt und säulenchromatographisch mit Dichlormethan-Methanol gereinigt: 80 mg Produkt.
MS (Cl) m/e = 488 (M⁺).

### Beispiel 8

### 4-Amino-8-chlor-7-(3-chlorbenzylamino)ethyl-2,3,3a,9b-tetrahydro-1Hcyclopenta[c]chinolin Dihydrochlorid

80 mg (0.16 mmol) 4-Amino-7-(*N*-*tert*-butoxycarbonyl-3-chlorbenzylamino)ethyl-8-chlor-2,3,3a,9b-tetrahydro-1H-cyclopenta[c]chinolin werden in 2.5 ml 4 M salzsaurem Dioxan 30 min bei Raumtemp. gerührt. Die flüchtigen Bestandteile werden i. Vak. abdestilliert: 90 mg Rückstand.
MS (Cl) m/e = 388 ([MH⁺ - 2 HCl]).

### Beispiel 9

### 4-Amino-7-(N-tert-butoxycarbonyl-3-chlorbenzylamino)-1,2,3,3a,7,8,9,10boctahydro-dicyclopenta[c,g]chinolin

### Ausgangsverbindungen

### 8-(Methoxycarbonylethenyl)-1,2,3,3a,5,9b-hexahydrocyclopenta[c]chinolin-4-on

Eine Lösung von 4.0 g (15.0 mmol) 8-Brom-1,2,3,3a,5,9b-hexahydrocyclopenta[c]chinolin-4-on (DE-Aktenzeichen: 198 06 348.2) in 150 ml DMF wird mit 2.7 ml (30.0 mmol) Acrylsäure-methylester, 4.2 ml (30 mmol) Triethylamin, 168 mg (0.75 mmol) Palladium(II)acetat und 457 mg (1.5 mmol) Tri-o-tolylphosphin 4 h auf 120 °C erhitzt. Nach Zugabe von 868 mg (0.75 mmol) Tetrakis(triphenylphosphin)palladium wird der Ansatz 18 h bei 120 °C gerührt. bei Raumtemp. mit Essigester verdünnt, mit Wasser gewaschen, getrocknet (Na₂SO₄) und i. Vak. eingeengt. Säulenchromatographie an Kieseigel mit Hexan-Essigester liefert 2.05 g Produkt.
¹H-NMR (CDCl₃): δ = 1.59- 1.80 (m, 3H), 2.14 (m, 2H), 2.34 (m, 1 H). 2.98 (td, 1 H), 3.29 (q, 1 H), 3.83 (s, 3H), 6.36 (d, 1 H), 6.81 (d, 1 H). 7.35 (dd, 1 H), 7.38 (d, 1H), 7.64 (d, 1H), 8.64 (br.s, 1 H).

### 8-(Methoxycarbonylethyl)-1,2,3,3a,5,9b-hexahydrocyclopenta[c]chinolin-4-on

2.73 g (10.1 mmol) 8-(Methoxycarbonylethenyl)-1,2,3,3a,5,9b-hexahydrocyclopenta[c]chinolin-4-on werden in 100 ml Essigester gelöst und nach Zugabe von 273 mg 10 proz. Pd-C und 0.1 ml Essigsäure 24 h in einer Wasserstoffatmosphäre gerührt. Nach Filtration wird das Filtrat eingeengt und säulenchromatographisch an Kieselgel mit Hexan-Essigester gereinigt: 2.7 g.
¹H-NMR (CDCl₃): δ = 1.57- 1.78 (m, 3H), 2.11 (m, 2H), 2.32 (m, 1 H). 2.62 (t, 2H), 2.90 (t, 2H), 2.95 (td, 1H), 3.24 (q, 1 H), 3.68 (s, 3H), 6.68 (d. 1H), 7.00 (dd, 1H), 7.04 (d, 1 H), 8.11 (br.s, 1 H).

### 8-(Carboxyethyl)-1,2,3,3a,5,9b-hexahydrocyclopenta[c]chinolin-4-on

Eine Lösung von 2.7 g (9.9 mmol) 8-(Methoxycarbonylethyl)-1,2,3,3a,5,9bhexahydrocyclopenta[c]chinolin-4-on in 30 ml THF und 50 mL Methano, wird mit 19.8 ml (19.8 mmol) 1 M NaOH-Lösung versetzt und 24 h bei Raumtemp. gerührt. Mit 10 proz. Schwefelsäure wird der Ansatz auf einen pH-Wert von 5 eingestellt und mit Essigester extrahiert. Die vereinigten Extrakte werden mit Wasser gewaschen. getrocknet (Na₂SO₄) und i. Vak. eingeengt.

Säuienchromatographie an Kieselgel mit Essigester-Hexan liefert 2.06 g Produkt.
¹H-NMR (D₆-DMSO): δ = 1.45 (pent, 1 H), 1.61 (m, 2H), 1.93 (m, 1 H), 1.99 (m, 1 H), 2.16 (m, 1 H), 2.50 (t, 2H), 2.75 (t, 2H), 2.80 (td, 1 H), 3.17 (q, 1 H), 6.77 (d, 1H), 6.98 (dd, 1 H), 7.06 (d, 1 H), 9.91 (br., 1 H), 12.00 (br., 1 H).

### 1,2,3,3a,7,8,9,10b-Octahydro-dicyclopenta[c,g]chinolin-4,7-dion

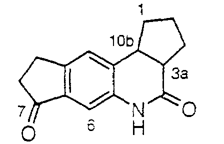

1.0 g (3.9 mmol) 8-(Carboxyethyl)-1,2,3,3a,5,9b-hexahydrocyclopenta[c]chinolin-4-on wird portionsweise zu 16 g Polyphosphorsäure gegeben, die vorher auf 120 °C erwärmt wurden. Nach 1 h bei 120 °C wird die Reaktionslösung auf Eiswasser gegeossen und mit Dichlormethan extrahiert. Die vereinigten Extrakte werden getrocknet (Na₂SO₄) und i. Vak. eingeengt. Säulenchromatographie des Rückstands an Kieslegel mit Hexan-Essigester liefert 400 mg Produkt.
¹H-NMR (CDCl₃): δ = 1.59- 1.83 (m, 3H), 2.12 (m, 2H), 2.42 (m, 1 H), 2.70 (m, 2H), 2.98 (td, 1 H), 3.09 (m, 2H), 3.31 (q, 1 H), 7.15 (s, 1 H), 7.32 (s, 1 H), 8.44 (br.s, 1 H).
Als Nebenprodukt entsteht 1,2,3,3a,8,9,10c -Octahydrodicyclopenta[c,f]chinolin-4,10-dion: 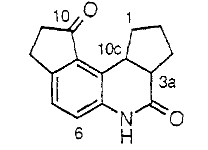 ¹H-NMR (CDCl₃): δ = 1.35 (m, 1 H), 1.77 (m, 2H), 2.12 (m, 1 H), 2.31 (m, 1 H), 2.56 (m, 1 H), 2.73 (m, 2H), 2.96 (td, 1 H), 3.10 (m, 2H), 4.23 (dt, 1 H), 7.03 (d, 1 H), 7.28 (d, 1 H), 8.95 (br.s, 1 H).

### 7-(N-tert-Butoxycarbonyl-3-chlorbenzylamino)-1,2,3,3a,7,8,9,10b-octahydrodicyclopenta[c,g]chinolin-4-on

Eine Lösung von 400 mg (1.66 mmol) 1,2,3,3a,7,8,9,10b-Octahydro-dicyclopenta[c,g]chinolin-4,7-dion in 50 ml 1,2-Dichlorethan und 50 ml THF wird mit 0.22 ml (1.82 mmol) 3-Chlorbenzylamin, 526 mg (2.49 mmol) Natrium-(triacetoxy)borhydrid und 0.01 ml (0.17 mmol) Essigsäure versetzt. Nach 24 h bei Raumtemp. werden 0.44 ml (3.6 mmol) 3-Chlorbenzylamin, 1.05 g (5.0 mmol) Natrium(triacetoxy)borhydrid und 0.02 ml (0.34 mmol) Essigsäure dazugegeben und weitere 24 h bei Raumtemp. gerührt. Die Reaktionslösung wird mit Essigester verdünnt, mit Wasser gewaschen, getrocknet (Na₂SO₄) und i. Vak. eingeengt. Säulenchromatographie an Kieselgel mit Hexan-Essigester liefert 360 mg 7-(3-Chlorbenzylamino)-1,2,3,3a,7,8,9,10b-octahydrodicyclopenta[c,g]chinolin-4-on.
Zu einer Lösung von 140 mg (0.26 mmol) 7-(3-Chlorbenzylamino)-1,2,3,3a,7,8,9,10b-octahydro-dicyclopenta[c,g]chinolin-4-on in 20 ml Dichlormethan werden 69 mg (0.32 mmol) Di-*tert*-butylkohlensäureanhydrid gegeben. Nach 24 h werden weitere 17 mg (0.08 mmol) Di-*tert*-butylkohlensäureanhydrid hinzugefügt, der Ansatz 24 h bei Raumtemp. gerührt, mit Dichlormethan verdünnt, mit Wasser gewaschen, getrocknet (Na₂SO₄) und i. Vak. eingeengt. Säulenchromatographie an Kieselgel mit Hexan-Essigester liefert 60 mg Produkt.
MS (FAB) m/e = 467 (M⁺).

### 7-(N-tert-Butoxycarbonyl-3-chlorbenzylamino)-1,2,3,3a,7,8,9,10b-octahydrodicyclopenta[c,g]chinolin-4-thion

Eine Lösung von 100 mg (0.21 mmol) 7-(*N*-*tert*-Butoxycarbonyl-3-chlorbenzylamino)-1,2,3,3a,7,8,9,10b-octahydro-dicyclopenta[c,g]chinolin-4-on und 96 mg (0.23 mmol) Lawessons Reagenz in 10 ml DME wird 2 h bei Raumtemp. gerührt. Nach Einengen wird der Rückstand säulenchromatographisch an Kieselgel mit Hexan-Essigester gereinigt: 70 mg Produkt.
MS (FAB) m/e = 483 (M⁺).

### 4-Amino-7-(N-tert-butoxycarbonyl-3-chlorbenzylamino)-1,2,3,3a,7,8,9,10boctahydro-dicyclopenta[c,g]chinolin

70 mg (0.15 mmol) 7-(*N*-*tert*-Butoxycarbonyl-3-chlorbenzylamino)-1,2,3,3a,7,8,9,10b-octahydro-dicyclopenta[c,g]chinolin-4-thion werden in 15 ml 7 M ammoniakalischem Methanol gelöst und 40 h bei Raumtemp. und 16 h bei 40 °C gerührt. Nach Einengen wird der Rückstand säulenchromatographisch an Kieselgel mit Dichlormethan-Methanol-Ammoniak gereinigt: 60 mg Produkt.
MS (ESI) m/e = 466 (M⁺).

### Beispiel 10

### 4-Amino-7-(3-chlorbenzylamino)-1,2,3,3a,7,8,9,10b-octahydrodicyclopenta[c,g]chchinolin Dihydrochlorid

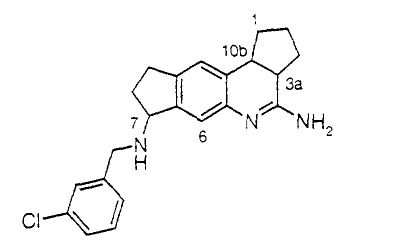

60 mg (0.13 mmol) 4-Amino-7-(*N*-*tert*-butoxycarbonyl-3-chlorbenzylamino)-1,2,3,3a,7,8,9,10b-octahydro-dicyclopenta[c,g]chinolin werden in 2 ml 4 M salzsaurem Dioxan 30 min bei Raumtemp. gerührt. Die flüchtigen Bestandteile werden i. Vak. abdestilliert: 60 mg Rückstand.
MS (ESI) m/e = 366 (M⁺ - 2 HCl).

## Patentansprüche

1. Verbindungen der Formel I, deren tautomere und isomere Formen und Salze 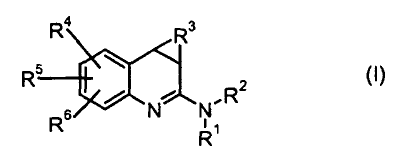 worin die Substituenten folgende Bedeutung haben:
R¹ und R² bedeuten:
Wasserstoff,
R³ bedeutet:
einen C₁₋₅-Alkylenrest,
R⁴ bedeutet:
C₁₋₄-Alkyl, substituiert mit NR¹⁴R¹⁵ oder
R⁴ und R⁵ bilden gemeinsam mit 2 benachbarten Kohlenstoffatomen einen fünfoder sechsgliedrigen Carbocyclus, der mit NR¹⁴R¹⁵ substituiert sein kann,
R⁵ und R⁶ bedeuten unabhängig voneinander:
a) Wasserstoff,
b) Halogen,
c) OR⁷,
d) C₁₋₄-Alkyl
e) CF₃,
f) OCF₃,
R⁷ bedeutet:
a) Wasserstoff,
b) C₁₋₆-Alkyl,
c) C6-10-Aryl, das gegebenenfalls mit Halogen oder C₁₋₄-Alkyl substituiert ist,
R²⁰ bedeutet:
a) C₁₋₆-Alkyl,
b) C6-10-Aryl, das gegebenenfalls mit Halogen oder C₁₋₄-Alkyl substituiert ist,
R¹⁴ und R¹⁵ bedeuten unabhängig voneinander:
a) Wasserstoff
b) CO₂R²⁰
c) C₁₋₆-Alkyl, das gegebenfalls substituiert ist mit Halogen, Hydroxy, C₁₋₄-Alkoxy, Nitro, Amino, C₁₋₆-Alkyl, Trifluormethyl, Carboxyl, Cyano, Carboxamido, C₃₋₇-Cycloalkyl, Indanyl, 1,2,3,4-Tetrahydronaphthyl, C₆₋₁₀-Aryl, 5- oder 6gliedrigen Heteroaryl mit 1 - 4 Stickstoff-, Sauerstoffoder Schwefelatomen, das mit Benzol anelliert sein kann, wobei der Arylund der Heteroarylrest mit Halogen, Hydroxy, C₁₋₄-Alkoxy, C₁₋₄-Alkyl, CF₃, NO₂, NH₂, N(C₁₋₄-Alkyl)₂ oder Carboxyl substituiert sein können,
oder
R¹⁴ und R¹⁵ bilden gemeinsam mit dem Stickstoffatom einen 5 - 7gliedrigen gesättigten Heterocyclus, der ein weiteres Sauerstoff-, Stickstoff- oder Schwefelatom enthalten und mit C₁₋₄-Alkyl oder einem gegebenenfalls mit Halogen substituierten Phenyl-, Benzyl- oder Benzoyl-Rest substituiert sein kann oder einen ungesättigten 5gliedrigen Heterocyclus, der 1 - 3 N-Atome enthalten und substituiert sein kann mit Phenyl, C₁₋₄-Alkyl, Halogen oder CH₂-OH.

2. Verbindungen gemäß Anspruch 1, worin R⁶ Wasserstoff oder Halogen ist.

3. Verbindungen gemäß Anspruch 1, worin R⁵ Wasserstoff bedeutet.

4. Verbindungen gemäß Anspruch 1, worin R⁴ und R⁵ gemeinsam mit zwei benachbarten Kohlenstoffatomen einen 5- oder 6gliedrigen Carbocyclus bilden, der mit NR¹⁴R¹⁵ substituiert ist.

5. 4-Amino-7-(N-tert-butoxycarbonyl-3-chlorbenzylamino)methyl-2,3,3a,9btetrahydro-1H-cyclopenta[c]chinolin
4-Amino-7-(3-chlorbenzylamino)methyl-2,3,3a,9b-tetrahydro-1Hcyclopenta[c]chinolin Dihydrochlorid
4-Amino-7-(N-tert-butoxycarbonyl-3-chlorbenzylamino)ethyl-2,3,3a,9btetrahydro-1H-cyclopenta[c]chinolin
4-Amino-7-(3-chlorbenzylamino)ethyl-2,3,3a,9b-tetrahydro-1Hcyclopenta[c]chinolin Dihydrochlorid
4-Amino-7-(N-tert-butoxycarbonyl-3-chlorbenzylamino)-1,2,3,3a,7,8,9,10boctahydro-dicyclopenta[c,g]chinolin
4-Amino-7-(3-chlorbenzylamino)-1,2,3,3a,7,8,9,10b-octahydrodicyclopenta[c,g]chinolin
4-Amino-7-[1-(*N*-*tert*-butoxycarbonyl-3-chlorbenzylamino)propyl]-2,3,3a,9btetrahydro-1H-cyclopenta[c]chinolin
4-Amino-7-[1-(3-chlorbenzylamino)propyl]-2,3,3a,9b-tetrahydro-1 H-cyclopenta[c]chinolin
4-Amino-7-(*N*-*tert*-butoxycarbonyl-3-chlorbenzylamino)ethyl-8-chlor-2,3,3a,9btetrahydro-1H-cyclopenta[c]chinolin
4-Amino-8-chlor-7-(3-chlorbenzylamino)ethyl-2,3,3a,9b-tetrahydro-1Hcyclopenta[c]chinolin Dihydrochlorid
gemäß Anspruch 1

6. Arzneimittel enthaltend eine Verbindung gemäß Anspruch 1 - 5 und einen pharmazeutisch üblichen Träger- und Hilfsstoff.

7. Verwendung der Verbindungen gemäß Anspruch 1 - 5 zur Herstellung eines Arzneimittels.

8. Verwendung der Verbindungen gemäß Anspruch 1 - 5 zur Herstellung eines Arzneimittels zur Behandlung einer Erkrankung, die durch NOS ausgelöst wird.

9. Verwendung nach Anspruch 8 zur Behandlung neurodegenerativer Erkrankungen.

10. Verfahren zur Herstellung einer Verbindung gemäß Anspruch 1 **dadurch gekennzeichnet, daß** man eine Verbindung der Formel (II) oder deren Salz 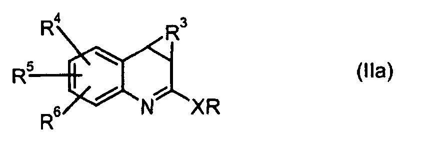 oder 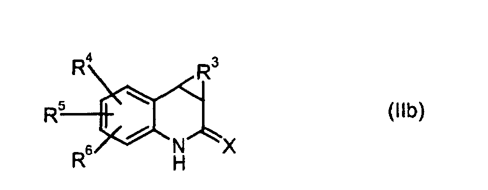 worin R³ bis R⁶ die Bedeutung nach Anspruch 1 haben, R Methyl oder Ethyl bedeutet und X = O oder S ist, mit Ammoniak, primären oder sekundären Aminen, Hydroxylamin und seinen Derivaten oder Hydrazin und seinen Derivaten umsetzt und gewünschtenfalls anschließend die Isomeren trennt und die Salze bildet.

11. Verbindungen der Formel IIb 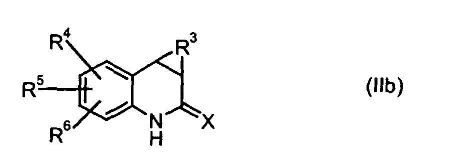 worin R³ bis R⁶ die Bedeutung nach Anspruch 1 haben und X = O oder S ist.

## Claims

1. Compounds of formula I, their tautomeric and isomeric forms and salts 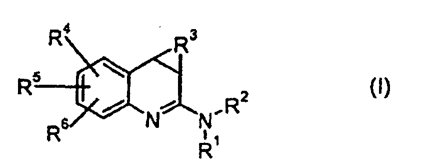 in which the substituents have the following meaning:
R¹ and R² mean:
hydrogen,
R³ means:
a C₁₋₅-alkylene radical,
R⁴ means:
C₁₋₄-alkyl substituted with NR¹⁴R¹⁵ or
R⁴ and R⁵ combine with 2 adjacent carbon atoms to form a five- or six-membered carbocycle which can be substituted with NR¹⁴R¹⁵,
R⁵ and R⁶ mean independently of one another:
a) hydrogen,
b) halogen,
c) OR⁷,
d) C₁₋₄-alkyl,
e) CF₃,
f) OCF₃,
R⁷ means:
a) hydrogen,
b) C₁₋₆-alkyl,
c) C₆₋₁₀-aryl, which is optionally substituted with halogen or C₁₋₄-alkyl,
R²⁰ means:
a) C₁₋₆-alkyl,
b) C₆₋₁₀-aryl, which is optionally substituted with halogen or C₁₋₄-alkyl,
R¹⁴ and R¹⁵ mean, independently of one another:
a) hydrogen,
b) CO₂R²⁰
c) C₁₋₆-alkyl, which optionally is substituted with halogen, hydroxy, C₁₋₄-alkoxy, nitro, amino, C₁₋₆-alkyl, trifluoromethyl, carboxyl, cyano, carboxamido, C₃₋₇-cycloalkyl, indanyl, 1,2,3,4-tetrahydronaphthyl, C₆₋₁₀-aryl, 5- or 6-membered heteroaryl with 1-4 nitrogen, oxygen or sulfur atoms, which can be annelated with benzene, where the aryl radical and the heteroaryl radical can be substituted with halogen, hydroxy, C₁₋₄-alkoxy, C₁₋₄-alkyl, CF₃, NO₂, NH₂, N(C₁₋₄-alkyl)₂ or carboxyl,
or R¹⁴ and R¹⁵ together with the nitrogen form a 5- to 7-membered saturated heterocycle, which can contain another oxygen, nitrogen or sulfur atom and can be substituted with C₁₋₄-alkyl or a phenyl, benzyl or benozyl radical that is optionally substituted with halogen, or an unsaturated 5-membered heterocycle, which can contain 1-3 N atoms and can be substituted with phenyl, C₁₋₄-alkyl, halogen or CH₂-OH.

2. Compounds according to claim 1, in which R⁶ is hydrogen or halogen.

3. Compounds according to claim 1, in which R⁵ is hydrogen.

4. Compounds according to claim 1, in which R⁴ and R⁵ together with two adjacent carbon atoms form a 5- or 6-membered carbocyclic compound, which is substituted with NR¹⁴R¹⁵,

5. 4-Amino-7-(N-tert-butoxycarbonyl-3- , chlorobenzylamino)methyl-2,3,3a,9b-tetrahydro-1H-cyclopenta[c]quinoline
4-amino-7-(3-chlorobenzylamino)methyl-2,3,3a,9b-tetrahydro-1H-cyclopenta[c]quinoline dihydrochloride
4-amino-7-(N-tert-butoxycarbonyl-3-chlorobenzylamino)ethyl-2,3,3a,9b-tetrahydro-1H-cyclopenta[c]quinoline
4-amino-7-(3-chlorobenzylamino)ethyl-2,3,3a,9b-tetrahydro-1H-cyclopenta[c]quinoline dihydrochloride
4-amino-7-(N-tert-butoxycarbonyl-3-chlorobenzylamino)-1,2,3,3a,7,8,9,10b-octahydrodicyclopenta[c,g]quinoline
4-amino-7-(3-chlorobenzylamino)-1,2,3,3a,7,8,9,10b-octahydrodicyclopenta[c,g]quinoline
4-amino-7-[1-(N-tert-butoxycarbonyl-3-chlorobenzylamino)propyl]-2,3,3a,9b-tetrahydro-1H-cyclopenta[c]quinoline
4-amino-7-[1-(3-chlorobenzylamino)propyl]-2,3,3a,9b-tetrahydro-1H-cyclopenta[c]quinoline
4-amino-7-(N-tert-butoxycarbonyl-3-chlorobenzylamino)ethyl-8-chloro-2,3,3a,9b-tetrahydro-1H-cyclopenta[c]quinoline
4-amino-8-chloro-7-(3-chlorobenzylamino)ethyl-2,3,3a,9b-tetrahydro-1H-cyclopenta[c]quinoline dihydrochloride
according to claim 1.

6. Pharmaceutical agent that contains a compound according to claims 1 to 5 and a pharmaceutically common vehicle and adjuvant.

7. Use of the compounds according to claims 1 to 5 for the production of a pharmaceutical agent.

8. Use of the compounds according to claims 1 to 5 for the production of a pharmaceutical agent for treating a disease which is triggered by NOS.

9. Use according to claim 8 for treating neurodegenerative diseases.

10. Process for the production of a compound according to claim 1, **characterized in that** a compound of formula (II) or its salt 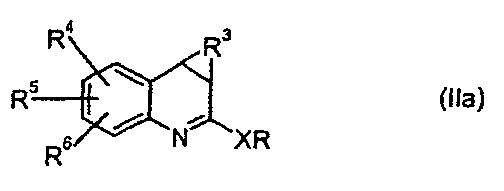 or 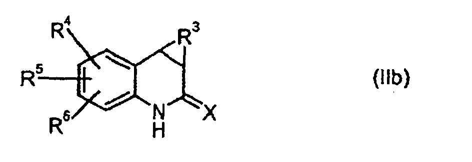 in which R³ to R⁶ have the meaning of claim 1, R means methyl or ethyl and X = O or S, is reacted with ammonia, primary or secondary amines, hydroxylamine and its derivatives or hydrazine and its derivatives, and optionally the isomers are then separated and the salts are formed.

11. Compounds of formula IIb 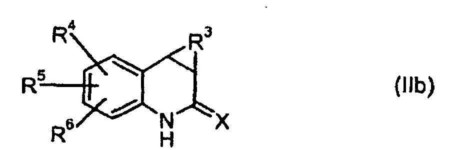 in which R³ to R⁶ have the meaning of claim 1, and X = O or S.

## Revendications

1. Composés de formule I, leurs formes tautomères et isomères et sels 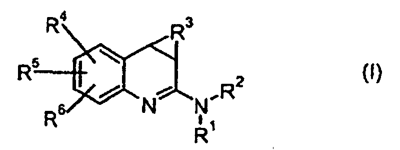 formule dans laquelle les substituants ont les significations suivantes :
R¹ et R² représentent :
un atome d'hydrogène,
R³ représente :
un radical alkylène en C₁₋₅,
R⁴ représente :
un groupe alkyle en C₁₋₄, substitué par NR¹⁴R¹⁵ ou
R⁴ et R⁵ forment ensemble, avec les 2 atomes de carbone voisins, un carbocycle à 5 ou 6 chaînons qui peut être substitué par NR¹⁴R¹⁵,
R⁵ et R⁶ représentent, indépendamment l'un de l'autre :
a) un atome d'hydrogène,
b) un atome d'halogène,
c) OR⁷,
d) un groupe alkyle en C₁₋₄,
e) CF₃,
f) OCF₃,
R⁷ représente :
a) un atome d'hydrogène,
b) un groupe alkyle en C₁₋₆,
c) un groupe aryle en C₆₋₁₀ qui est éventuellement substitué par un atome d'halogène ou par un groupe alkyle en C₁₋₄,
R²⁰ représente :
a) un groupe alkyle en C₁₋₆,
b) un groupe aryle en C₆₋₁₀ qui est éventuellement substitué par un atome d'halogène ou par un groupe alkyle en C₁₋₄,
R¹⁴ et R¹⁵ représentent indépendamment l'un de l'autre :
a) un atome d'hydrogène,
b) CO₂R²⁰,
c) un groupe alkyle en C₁₋₆ qui est éventuellement substitué par un atome d'halogène ou par un, groupe hydroxy, alcoxy en C₁₋₄, nitro, amino, alkyle en C₁₋₆, trifluorométhyle, carboxy, cyano, carboxamido, cycloalkyle en C₃₋₇, indanyle, 1,2,3,4-tétrahydronaphtyle, aryle en C₆₋₁₀, hétéroaryle à 5 ou 6 chaînons ayant de 1 à 4 atomes d'azote, d'oxygène ou de soufre, qui peut être soudé à un noyau benzénique, le radical aryle et le radical hétéroaryle pouvant être substitués par un atome d'halogène ou par un groupe hydroxy, alcoxy en C₁₋₄, alkyle en C₁₋₄, CF₃, NO₂, NH₂, N[alkyle(C₁₋₄)]₂ ou carboxy,
ou
R¹⁴ et R¹⁵ forment ensemble, avec l'atome d'azote, un hétérocycle saturé à 5-7 chaînons qui peut contenir un autre atome d'oxygène, d'azote ou de soufre et être substitué par un groupe alkyle en C₁₋₄ ou par un radical phényle, benzyle ou benzoyle éventuellement substitué par un atome d'halogène, ou un hétérocycle insaturé à 5 chaînons qui peut contenir 1-3 atomes d'azote et être substitué par un atome d'halogène ou par un groupe phényle, alkyle en C₁₋₄ ou CH₂OH.

2. Composés selon la revendication 1, dans lesquels R⁶ est un atome d'hydrogène ou d'halogène.

3. Composés selon la revendication 1, dans lesquels R⁵ représente un atome d'hydrogène.

4. Composés selon la revendication 1, dans lesquels R⁴ et R⁵ forment ensemble, avec les 2 atomes de carbone voisins, un carbocycle à 5 ou 6 chaînons qui est substitué par NR¹⁴R¹⁵.

5. 4-amino-7-(N-tert-butoxycarbonyl-3-chlorobenzylamino)méthyl-2,3,3a,9b-tétrahydro-1H-cyclopenta[c]-quinoléine,
dichlorhydrate de 4-amino-7-(3-chlorobenzylamino)-méthyl-2,3,3a,9b-tétrahydro-1H-cyclopenta[c]-quinoléine,
4-amino-7-(N-tert-butoxycarbonyl-3-chlorobenzylamino)-éthyl-2,3,3a,9b-tétrahydro-1H-cyclopenta[c]-quinoléine,
dichlorhydrate de 4-amino-7-(3-chlorobenzylamino)éthyl-2,3,3a,9b-tétrahydro-1H-cyclopenta[c]quinoléine,
4-amino-7-(N-tert-butoxycarbonyl-3-chlorobenzylamino)-1,2,3,3a,7,8,9,10b-octahydrodicyclopenta[c,g]-quinoléine,
4-amino-7-(3-chlorobenzylamino)-1,2,3,3a,7,8,9,10b-octahydrodicyclopenta[c,g]quinoléine,
4-amino-7-[1-(N-tert-butoxycarbonyl-3-chlorobenzylamino)propyl]-2,3,3a,9b-tétrahydro-1H-cyclopenta[c]quinoléine,
4-amino-7-[1-(3-chlorobenzylamino)propyl]-2,3,3a,9b-tétrahydro-1H-cyclopenta[c]quinoléine,
4-amino-7-(N-tert-butoxycarbonyl-3-chlorobenzylamino)-éthyl-8-chloro-2,3,3a,9b-tétrahydro-1H-cyclopenta-[c]quinoléine,
dichlorhydrate de 4-amino-8-chloro-7-(3-chlorobenzylamino)éthyl-2,3,3a,9b-tétrahydro-1H-cyclopenta[c]-quinoléine,
selon la revendication 1.

6. Médicament contenant un composé selon l'une quelconque des revendications 1 à 5 et un véhicule et un adjuvant pharmaceutiquement usuels.

7. Utilisation des composés selon l'une quelconque des revendications 1 à 5, pour la fabrication d'un médicament.

8. Utilisation des composés selon l'une quelconque des revendications 1 à 5, pour la fabrication d'un médicament destiné au traitement d'une maladie qui est déclenchée par une NOS (monoxyde d'azote synthase).

9. Utilisation selon la revendication 8, pour le traitement de maladies neurodégénératives.

10. Procédé pour la préparation d'un composé selon la revendication 1, **caractérisé en ce qu'**on fait réagir un composé de formule (II) 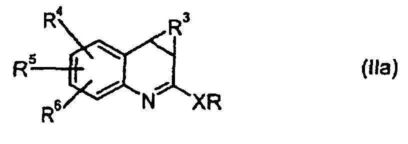 ou 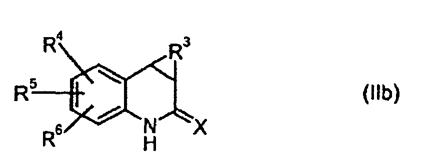 formules dans lesquelles R³ à R⁶ ont les significations selon la revendication 1, R représente le groupe méthyle ou éthyle et X est O ou S, ou un sel d'un tel composé, avec de l'ammoniac, des amines primaires ou secondaires, de l'hydroxylamine et ses dérivés ou de l'hydrazine et ses dérivés, et, si on le désire, ensuite on sépare les isomères et on forme les sels.

11. Composés de formule IIb 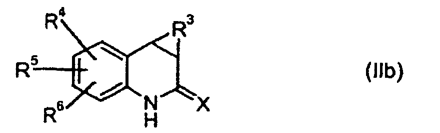 dans laquelle R³ à R⁶ ont les significations selon la revendication 1 et X est O ou S.
